(19) Europäisches Patentamt
European Patent Office
Office européen des brevets

(11) Veröffentlichungsnummer: **0 083 792**
**A2**

(12) # EUROPÄISCHE PATENTANMELDUNG

(21) Anmeldenummer: 82112018.5

(22) Anmeldetag: 27.12.82

(51) Int. Cl.³: C 07 C 93/197
C 07 D 295/08, C 07 D 233/60
C 07 D 303/22, A 01 N 53/00

(30) Priorität: 09.01.82 DE 3200484

(43) Veröffentlichungstag der Anmeldung:
20.07.83 Patentblatt 83/29

(84) Benannte Vertragsstaaten:
AT BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Fuchs, Rainer, Dr.
Roeberstrasse 8
D-5600 Wuppertal 1(DE)

(72) Erfinder: Hammann, Ingeborg, Dr.
Lutherstrasse 22
D-4330 Mülheim/Ruhr(DE)

(72) Erfinder: Stendel, Wilhelm, Dr.
In den Birken 55
D-5600 Wuppertal 1(DE)

(54) **Aminoalkylester, Verfahren zu ihrer Herstellung und ihre Verwendung in Schädlingsbekämpfungsmitteln.**

(57) Die vorliegende Erfindung betrifft:
1. neue Aminoalkylester der Formel (I)

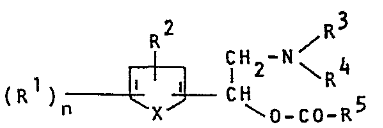

(I)

in welcher
$R^1$ - $R^5$, X und n die in der Beschreibung angegebene Bedeutung haben. Sie werden hergestellt, indem man Aminoethanole der Formel II

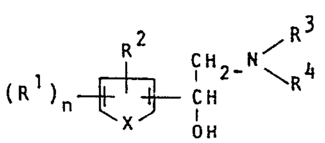

(II)

in welcher
$R^1$, $R^2$, $R^3$, $R^4$ n und X die in der Beschreibung angegebenen Bedeutungen haben,
oder deren Salze, mit Carbonsäuren der Formel III

$R^5COOH$ (III)

in welcher
$R^5$ die oben angegebene Bedeutung hat,
oder mit Reaktionsfähigen Derivaten derselben, gegebenenfalls in Gegenwart von Säureakzeptoren, gegebenenfalls in Gegenwart von Katalysatoren und gegebenenfalls in Gegenwart von Verdünnungsmitteln umsetzt. Erfindung betrifft ferner Zwischenprodukte zur Herstellung der Verbindungen der Formel I.

Die neuen Aminoalkylester der Formel (I) zeichnen sich durch hohe pestizide, insbesondere insektizide Wirksamkeit aus.

EP 0 083 792 A2

0083792

BAYER AKTIENGESELLSCHAFT 5090 Leverkusen, Bayerwerk
Zentralbereich
Patente, Marken und Lizenzen Rt/Mai
Ia

# Aminoalkylester, Verfahren zu ihrer Herstellung und ihre Verwendung in Schädlingsbekämpfungsmitteln.

Die vorliegende Erfindung betrifft neue Aminoalkylester, ein Verfahren zu ihrer Herstellung und ihre Verwendung in Schädlingsbekämpfungsmitteln, insbesondere in Insektiziden.

Es ist bekannt, daß bestimmte Carbonsäureester, wie z.B. 3-(2,2-Dimethylvinyl)-2,2-dimethyl-cyclopropancarbonsäure-(3,4,5,6-tetrahydrophthalimido-methyl)-ester, insektizide Eigenschaften aufweisen (vergleiche US-PS 3 268 398). Die Wirkung dieser Verbindungen ist jedoch, insbesondere bei niedrigen Wirkstoffkonzentrationen und Aufwandmengen nicht immer zufriedenstellend.

Die vorliegende Erfindung betrifft:

1. neue Aminoalkylester der Formel (I)

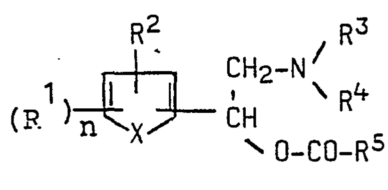

(I)

in welcher

$R^1$ und $R^2$ gleich oder verschieden sind und für Wasserstoff, gegebenenfalls substituierte Reste aus der Reihe Alkyl, Alkenyl, Alkinyl, Alkoxy, Alkylthio, Aryl, Aralkyl und Aryloxy sowie für Halogen, Nitro und Cyano stehen, oder

$R^1$ und $R^2$ zusammen für gegebenenfalls substituiertes Alkylen oder Alkylendioxy stehen,

n für 1 oder 2 steht

$R^3$ und $R^4$ gleich oder verschieden sind und für Wasserstoff oder gegebenenfalls substituierte Reste aus der Reihe Alkyl, Alkenyl, Cycloalkyl, Aryl oder Aralkyl stehen, oder

$R^3$ und $R^4$ zusammen mit dem Stickstoff an den sie gebunden sind, für gegebenenfalls substituiertes Pyrrolidin-1-yl, Piperidin-1-yl, Morpholin-4-yl, Imidazol-1-yl, Pyrazol-1-yl, Pyrimidin-1-yl, Imidazolidin-1-yl, Piperazin-1-yl oder Pyryzolidin -1-yl stehen,

$R^5$ für einen in der Säurekomponente von Pyrethroiden verwendbaren Rest steht, und

X für -O-, -S-, -NH-, -CH=CH-, -CF=CF-, -CH=N-,

steht.

2. ein Verfahren zur Herstellung der neuen Verbindungen der Formel I

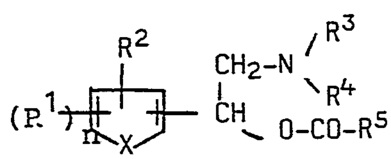

(I)

in welcher

$R^1$ und $R^2$ gleich oder verschieden sind und für Wasserstoff, gegebenenfalls substituierte Reste aus der Reihe Alkyl, Alkenyl, Alkinyl, Alkoxy, Alkylthio, Aryl, Aralkyl und Aryloxy sowie für Halogen, Nitro und Cyano stehen, oder

$R^1$ und $R^2$ zusammen für gegebenenfalls substituiertes Alkylen oder Alkylendioxy stehen,

n für 1 oder 2 steht,

$R^3$ und $R^4$ gleich oder verschieden sind und für Wasserstoff oder gegebenenfalls substituierte Reste aus der Reihe Alkyl, Alkenyl, Cycloalkyl, Aryl und Aralkyl stehen, oder

$R^3$ und $R^4$ zusammen mit dem Stickstoffatom, an das sie gebunden sind für gegebenenfalls substituiertes Pyrrolidin-1-yl, Piperidin-1-yl, Morpholin-4-yl, Imidazol-1-yl, Pyrazol-1-yl, Pyrimidin-1-yl, Imidazolin-1-yl, Piperazin-1-yl oder Pyrazolidin-1-yl stehen,

$R^5$ für einen in der Säurekomponente von Pyrethroiden verwendbaren Rest steht und

X für -O-, -S-, -NH-, -CH=CH-, -CF=CF-, -CH=N-, steht,

dadurch gekennzeichnet daß man Aminoethanole der Formel II

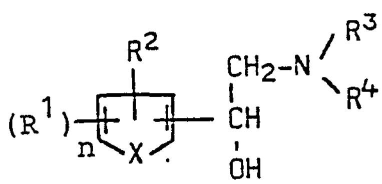

(II)

in welcher

n, $R^1$, $R^2$, $R^3$, $R^4$, und X die oben angegebene Bedeutung haben,
oder deren Salze,

mit Carbonsäuren der Formel III

$$R^5COOH \qquad (III)$$

in welcher

$R^5$ die oben angegebene Bedeutung hat,

oder mit reaktionsfähigen Derivaten derselben, gegebenenfalls in Gegenwart von Säureakzeptoren, gegebenenfalls in Gegenwart von Katalysatoren und gegebenenfalls in Gegenwart von Verdünnungsmitteln umsetzt;

3. Aminoethanole der Formel II b

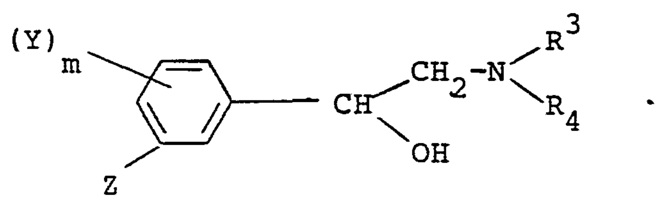

(IIb)

in welcher

Z für Fluor, Brom oder gegebenenfalls substituiertes Aryloxy steht,

Y für Wasserstoff oder Fluor,

m für 1 - 4 steht,
$R^3$ und $R^4$ die oben angegebene Bedeutung haben.

4. Ein Verfahren zur Herstellung der neuen Verbindungen der Formel (II), dadurch gekennzeichnet, daß man Oxacyclopropane der Formel IV b

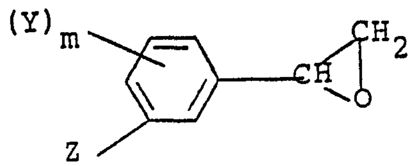

(IV b)

in welcher
Y, Z und m die oben angegebene Bedeutung haben,

mit den Aminen der Formel V

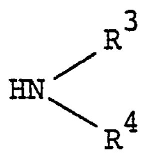

(V)

in welcher

$R^3$ und $R^4$ die oben angegebene Bedeutung haben,

gegebenenfalls in Gegenwart von Verdünnungsmitteln und/oder gegebenenfalls in Gegenwart eines Katalysators bei Temperaturen zwischen 0 und 100°C umsetzt;

5. Oxacyclopropane der Formel IV b

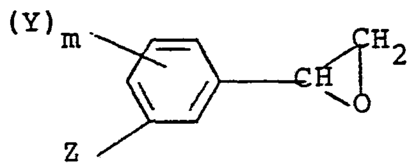

(IV b)

in welcher

Z für Fluor, Brom oder gegebenenfalls substituiertes Aryloxy steht,
Y für Wasserstoff oder Fluor steht,
m für 1-4 steht,

6. ein Verfahren zur Herstellung der neuen Verbindungen der Formel (IV b), dadurch gekennzeichnet, daß man Aldehyde der Formel VI

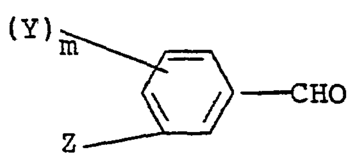

(VI)

in welcher
Z, Y und m die oben angegebene Bedeutung haben,

mit Trimethylsulfoniumhalogeniden der Formel VII

$$(H_3C)_3-S^{\oplus}\ Hal^{\ominus} \quad (VII)$$

in welcher

Hal für Chlor, Brom oder Iod steht,

gegebenenfalls in einer Schutzgasatmosphäre, gegebenenfalls in Gegenwart von Säureakzeptoren und gegebenenfalls in Gegenwart von Verdünnungsmitteln bei Temperaturen zwischen lo und 5o°C umsetzt.

Die neuen Aminoalkylester der Formel (I) zeichnen sich durch hohe pestizide, insbesondere insektizide Wirksamkeit aus.

Ueberraschenderweise zeigen die erfindungsgemäßen Verbindungen der Formel (I) eine erheblich höhere insektizide Wirkung als die aus dem Stand der Technik bekannte Verbindung 3-(2,2-Dimethylvinyl)-2,2-dimethyl-cyclopropancarbonsäure-(3,4,5,6-tetrahydrophthalimido-methyl)-ester, welche chemisch und wirkungsmäßig eine naheliegende Verbindung ist.

Die Erfindung betrifft vorzugsweise Verbindungen der Formel (I), in welcher

$R^1$ und $R^2$ gleich oder verschieden sind und für Wasserstoff, gegebenenfalls substituierte Reste aus der Reihe Alkyl, Alkoxy und Alkylthio mit jeweils 1 bis 4 Kohlenstoffatomen im Alkylteil, für Alkenyl oder Alkinyl mit jeweils 2 bis 4 Kohlenstoffatomen, Aryl oder Aryloxy mit jeweils 6 bis lo Kohlenstoffatomen im Arylteil, Aralkyl mit 6 bis lo Kohlenstoffatomen im Arylteil und 1 bis 4 Kohlenstoffatomen im Alkylteil sowie für Halogen, Nitro oder Cyano

stehen;
als Substituenten kommen vorzugsweise in Frage: Halogene, wie Fluor, Chlor oder Brom, Alkyl, Alkoxy oder Alkylthio mit jeweils 1 bis 4 Kohlenstoffatomen im Alkylteil, oder

$R^1$ und $R^2$ zusammen für gegebenenfalls durch Fluor und/oder Chlor und/oder Brom substituiertes Alkylen oder Alkylendioxy mit jeweils 2 bis 4 Kohlenstoffatomen stehen,

n für 1 oder 2 steht,

$R^3$ und $R^4$ gleich oder verschieden sind und für Wasserstoff oder gegebenenfalls substituierte Reste aus der Reihe Alkyl mit 1 bis 16 Kohlenstoffatomen, Alkenyl mit 2 bis 4 Kohlenstoffatomen, Cycloalkyl mit 3 bis 7 Kohlenstoffatomen, Aryl mit 6 bis 10 Kohlenstoffatomen oder Aralkyl mit 6 bis lo Kohlenstoffatomen im Arylteil und 1 bis 4 Kohlenstoffatomen im Alkylteil stehen;
als Substituenten kommen vorzugsweise in Frage: Halogene, wie Fluor, Chlor oder Brom oder Alkyl mit 1 bis. 4 Kohlenstoffatomen, oder

$R^3$ und $R^4$ zusammen mit dem Stickstoffatom, an das sie gebunden sind, für gegebenenfalls durch $C_1$-$C_4$-Alkyl substituiertes Pyrrolidin-1-yl, Piperidin-1-yl, Morpholin-4-yl, Imidazol-1-yl, Pyrazol-1-yl, Pyrimidin-1-yl, Imidazolidin-1-yl, Piperazin-1-yl oder Pyrazolidin-1-yl stehen,

$R^5$ für 2,2,3,3-Tetramethylcyclopropyl oder für den Rest

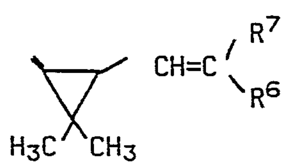

steht, worin

$R^6$ für Fluor, Chlor, Brom, gegebenenfalls halogen-substituiertes $C_1$-$C_4$-Alkyl oder für gegebenenfalls durch Halogen und /oder durch gegebenenfalls halogen-substituierte Reste aus der Reihe $C_1$-$C_4$-Alkyl, $C_1$-$C_4$-Alkoxy, $C_1$-$C_4$-Alkylthio oder $C_1$-$C_2$-Alkylendioxy substituiertes Phenyl steht, und

$R^7$ für Wasserstoff, Fluor, Chlor, Brom oder für gegebenenfalls halogen-substituiertes $C_1$-$C_4$-Alkyl steht, oder worin

$R^6$ und $R^7$ zusammen für $C_2$-$C_5$-Alkandiyl stehen;

in welcher weiter

$R^5$ vorzugsweise für den Rest

$-CH-R^9$
$R^8$

steht, worin

$R^8$ für Isopropyl oder Cyclopropyl steht und

$R^9$ für gegebenenfalls durch Halogen und/oder durch gegebenenfalls halogen-substituierte Reste aus der Reihe $C_1$-$C_4$-Alkyl, $C_1$-$C_4$-Alkoxy, $C_1$-$C_4$-Alkylthio oder $C_1$-$C_2$-Alkylendioxy substituiertes Phenyl steht;

in welcher weiter

$R^5$ vorzugsweise für den Rest

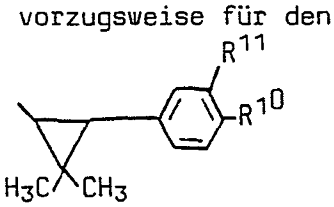

steht, worin

$R^{10}$ für einfach oder mehrfach, gleich oder verschieden halogensubstituierte $C_1$-$C_4$-Alkoxy- oder $C_1$-$C_4$-Alkylthioreste steht, und

$R^{11}$ für Wasserstoff oder Halogen steht oder

$R^{10}$ und $R^{11}$ gemeinsam für einfach oder mehrfach, gleich oder verschieden halogen-substituiertes Alkylendioxy stehen;

in welcher weiter

$R^5$ vorzugsweise für den Rest

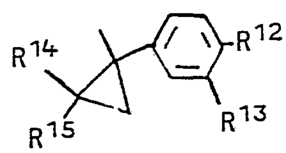

steht, worin

$R^{12}$ für Wasserstoff, Halogen, Cyano,Nitro, Amino oder für einen gegebenenfalls halogen-substituierten Rest aus der Reihe $C_1$-$C_4$-Alkyl, $C_1$-$C_4$-Alkoxy, $C_1$-$C_4$-Alkylthio steht, und

$R^{13}$ für Wasserstoff, Halogen, Methyl oder Methoxy steht oder

$R^{12}$ und $R^{13}$ zusammen für gegebenenfalls halogen-substituiertes $C_1$-$C_2$-Alkylendioxy stehen;

Die Erfindung betrifft vorzugsweise Verbindungen der Formel I in welcher

X für Sauerstoff, Stickstoff oder Schwefel steht, für CH=N- steht oder für -CH=CH- steht, wobei der dann gebildete Phenylring ein- bis dreifach durch Fluor, Chlor oder Brom substituiert sein kann.

Ganz besonders bevorzugt sind die Verbindungen der Formel (I), in welcher

$R^1$ und $R^2$ gleich oder verschieden sind und für Wasserstoff, Methyl, Ethyl, n-Propyl, iso-Propyl, n-Butyl, iso-Butyl, sec-Butyl, tert-Butyl, Methoxy, Ethoxy, n-Propoxy, iso-Propoxy, Trifluormethoxy, 1-Propen-3-yl, Benzyl, Phenoxy, 4-Fluorphenoxy, 4-Chlorphenoxy, 4-Bromphenoxy, Fluor, Chlor oder Brom stehen, oder

$R^1$ und $R^2$ zusammen für Trifluorethylendioxy, Difluormethylendioxy oder Ethylendioxy stehen,

$R^3$ und $R^4$ gleich oder verschieden sind und für Wasserstoff, Methyl, Ethyl, n-Propyl, iso-Propyl, n-Butyl, iso-Butyl, sec.-Butyl, n-Undecyl, n-Dodecyl, 1-Propen-3-yl, Cyclopropyl, Cyclopentyl, Cyclohexyl, Benzyl, Phenylethyl, 4-Chlorbenzyl, 4-Methylbenzyl oder 4-Brombenzyl stehen, oder

$R^3$ und $R^4$ zusammen mit dem Stickstoff an das sie gebunden sind, für Morpholin-4-yl, Imidazol-1-yl, Piperidin-1-yl, Piperazin-1-yl, 4-Methyl-piperazin-1-yl, 4-Ethyl-piperazin-1-yl, 4-n-Propyl-piperazin-1-yl oder 4-iso-Propyl-piperazin-1-yl stehen,

$R^5$ für 2,2,3,3-Tetramethylcyclopropyl oder für den Rest

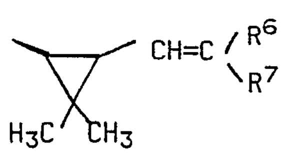

steht, worin

$R^6$ für Fluor, Chlor, Brom, Methyl, 4-Chlorphenyl, Trifluormethyl, 4-Bromphenyl, 4-Methylphenyl oder Phenyl steht,

$R^7$ für Wasserstoff, Chlor, Brom oder Methyl steht, oder

$R^6$ und $R^7$ zusammen für Ethandiyl oder Butandiyl stehen;

in welcher weiter

$R^5$ für den Rest

$-CH-R^9$
$\;\;\;R^8$

steht, worin

$R^8$ für Isopropyl oder Cyclopropyl steht und

$R^9$ für 4-Trifluormethoxyphenyl, 4-Difluormethoxyphenyl, 4-Chlorphenyl, 4-Methylphenyl, 4-tert-Butylphenyl, 4-Trifluormethyl-phenyl, 4-Methoxy-phenyl, Phenyl, 3-Brom-4-trifluormethoxyphenyl, 4-Trifluormethylthiophenyl, 3,4-Trifluorethylendioxy-phenyl, 3,4-Difluormethylendioxy-phenyl, 3,4-Methylendioxyphenyl oder 4-Chlortrifluorethoxy-phenyl steht;

in welcher weiter

$R^5$ für den Rest

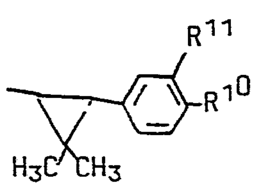

steht, worin

$R^{10}$ für Chlortrifluorethoxy, Tetrafluorethoxy, Chlordifluormethoxy, Trifluormethoxy, Trifluormethylthio und Difluormethoxy steht, und

$R^{11}$ für Wasserstoff, Chlor oder Brom steht,

$R^{10}$ und $R^{11}$ gemeinsam für Trifluorethylendioxy oder Difluormethylendioxy stehen;

in welcher weiter

$R^5$ für den Rest

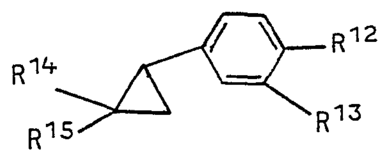

steht, worin

$R^{12}$ für Fluor,Chlor, Brom, Methoxy, Ethoxy, Trifluormethoxy, 2-Chlor-1,1,2-trifluorethoxy oder 1,1,2,2-Tetrafluorethoxy steht oder worin

$R^{13}$ für Wasserstoff steht und

$R^{12}$ und $R^{13}$ zusammen für Methylendioxy oder Difluormethylendioxy stehen;

$R^{14}$ und $R^{15}$ für Chlor stehen; und

X für -CH=CH- steht, wobei der so gebildete Phenylring dreifach durch Fluor substituiert sein kann.

Außerdem sind ganz besonders bevorzugt die Verbindungen der Formel (I), in welcher

$R^1$ und $R^2$ gleich oder verschieden sind und für Wasserstoff, Methyl, Ethyl, n-Propyl, iso-Propyl, tert-Butyl, Phenoxy, Chlor oder Brom stehen,

$R^3$, $R^4$ und $R^5$ die gleichen Bedeutungen haben, wie sie oben bei der Definition der entsprechenden Gruppen als ganz besonders bevorzugt angegeben sind, und

X für Sauerstoff, Stickstoff, Schwefel oder -CH=N- steht.

Eine besondere Variante (a) des unter (2) dargelegten Verfahrens zur Herstellung der neuen Verbindungen der Formel (I) - 'Verfahren (2 a)' - ist dadurch gekennzeichnet, daß man als reaktionsfähige Derivate der Carbonsäuren der Formel (III) Carbonsäurechloride der Formel III a

$$R^5\text{-CO-Cl} \qquad \text{(III a)}$$

in welcher

$R^5$ die unter (1) angegebene Bedeutung hat,

mit Aminoethanolen der Formel (II) (oben) gegebenenfalls in Gegenwart von Säureakzeptoren, gegebenenfalls in Gegenwart von Katalysatoren und gegebenenfalls unter Verwendung von Verdünnungsmitteln umsetzt.

Als weitere reaktionsfähige Derivate der Carbonsäure der Formel (III) sind deren Niederalkylester zu nennen, welche mit Aminoethanolen der Formel (II) nach üblichen Methoden zu Verbindungen der Formel (I) umgesetzt werden.

Eine weitere Variante (b) des unter (2) dargelegten Verfahrens zur Herstellung der neuen Verbindungen der Formel (I) - 'Verfahren 2 b' - ist dadurch gekennzeichnet, daß man Carbonsäurechloride der Formel III a (oben) mit Hydrohalogeniden der Aminoethanole der Formel II a

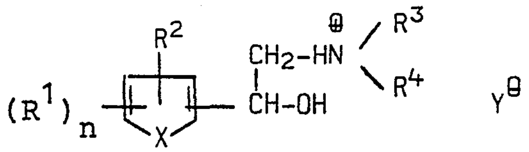

(II a)

in welcher

$R^1$, $R^2$, $R^3$, $R^4$, n und X die unter (1) angegebenen Bedeutungen haben und

Y für Chlor oder Brom steht,

gegebenenfalls in Gegenwart von Säureakzeptoren, gegebenenfalls in Gegenwart von Katalysatoren und gegebenenfalls unter Verwendung von Verdünnungsmitteln umsetzt.

Verwendet man als Ausgangsstoffe beispielsweise 1-(3-Phenoxy-phenyl)-2-methylamino-ethanol bzw. das entsprechende Hydrochlorid und 3,3-Dichlorvinyl-2,2-dimethyl-cyclopropancarbonsäurechlorid, so können die bei den Verfahrensvarianten (a) und (b) ablaufenden Reaktionen durch folgende Formelschemata skizziert werden:

(2 a)

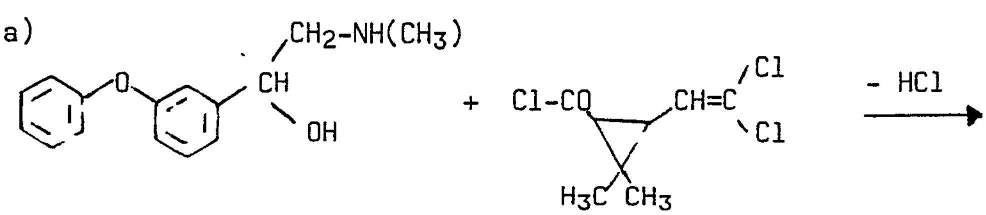

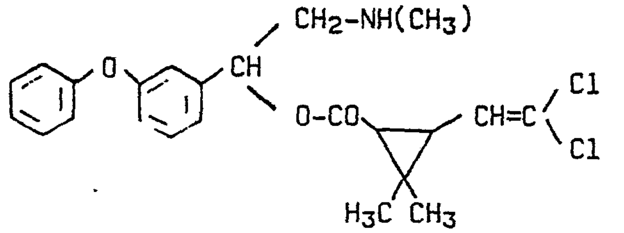

(2 b)

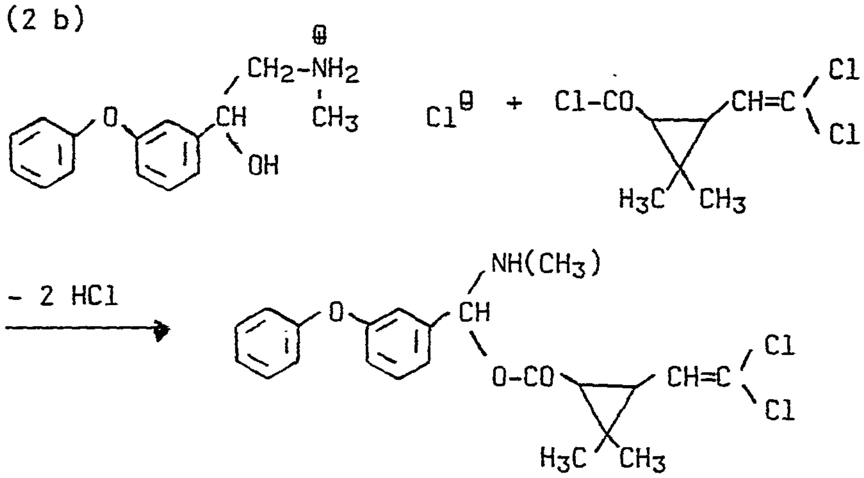


Das Verfahren zur Herstellung der neuen Verbindungen der Formel (I) wird in allen Varianten vorzugsweise unter Verwendung von Verdünnungsmitteln durchgeführt.

Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, Ketone wie Aceton, Methylethyl-, Methylisopropyl- und Methylisobutylketon, Ester wie Essigsäuremethylester und -ethylester, Nitrile, wie z.B. Acetonitril und Propionitril. Amide, wie z.B.Dimethylformamid, Dimethylacetamid und N-Methyl-pyrrolidon, sowie Dimethylsulfoxid, Tetramethylensulfon und Hexamethylphoshorsäuretriamid.

Als Säureakzeptoren für das Verfahren (2) können die üblichen Säurebindemittel verwendet werden. Als Beispiele seien genannt: Alkalihydroxide, wie z.B. Natrium- und Kaliumhydroxid, Alkalicarbonate, wie z.B. Natrium- und Kaliumcarbonat, Alkalialkoholate, wie z.B. Natrium- und Kaliummethylat und -ethylat, ferner aliphatische und aromatische, auch heterocylcische Amine, wie z.B. Triethyl- und Trimethylamin, N,N-Dimethyl-anilin, N,N-Dimethyl-benzylamin, Pyridin, Diazabicyclooctan, Diazabicyclononan und Diazabicycloundecen.

Die Reaktionstemperatur wird bei Verfahren (2) zwischen 0 und 15o°C, bei den Varianten (a) und (b) vorzugsweise zwischen lo und 12o°C gehalten. Das Verfahren wird im allgemeinen bei Normaldruck durchgeführt.

Zur Durchführung von Verfahren (2) - Varianten (a) und (b) - werden angenähert äquimolare Mengen der Ausgangsstoffe, gegebenenfalls zusammen mit Säureakzeptoren bzw. Katalysatoren, in geeigneten Verdünnungsmitteln zusammengegeben und die Reaktionsgemische werden mehrere Stunden gerührt. Zur Aufarbeitung, welche nach üblichen Methoden erfolgen kann, wird gegebenenfalls mit Wasser und/oder einem mit Wasser nicht mischbaren organischen Lösungsmittel verdünnt, die organische Phase abgetrennt, mit Wasser gewaschen, getrocknet und filtriert. Das Lösungsmittel wird unter vermindertem Druck sorgfältig abdestilliert, wobei das Rohprodukt als Rückstand verbleibt.

Die als Ausgangsstoffe zu verwendenden Aminoethanole sind durch die Formel (II), die entsprechenden Hydrohalogenide durch die Formel (II a) definiert. Vorzugsweise haben darin $R^1$, $R^2$, $R^3$, $R^4$, n und X die gleichen Bedeutungen, wie sie bei der Definition der entsprechenden Gruppen in Formel (I) als bevorzugt angegeben sind und Y steht für Chlor oder Brom.

Die Hydrohalogenide der Formel (II a) erhält man aus den entsprechenden Aminoethanolen der Formel (II) nach üblichen Methoden, beispielsweise durch Umsetzung mit den entsprechenden Halogenwasserstoffen, gegebenenfalls unter Verwendung eines Verdünnungsmittels, wie z.B. Toluol bei Temperaturen zwischen 0 und 100°C. Das Produkt kann ohne weitere Reinigung in die nächste Reaktion eingesetzt werden.

Die als Ausgangsprodukte zu verwendenden Aminoethanole der Formel (II) sind teilweise bekannt (vergleiche z.B. DE-OS 2 628 42o und US-PS 4 o38 4o9).
Die Aminomethanole der Formel II b sind neu.

Bevorzugt steht bei den neuen Aminomethanolen der Formel II b

Z für Fluor, Brom, Phenoxy oder Fluorphenoxy
Y für Fluor
m steht bevorzugt für 4 wenn Z für Fluor steht.

Als Beispiele für die Verbindungen der Formel (IIb) seien genannt:

1-(3-Brom-4-fluorphenyl)-2-dimethylamino-, 1-(3-Phenoxy-4-fluorphenyl)-2-(2-propylamino)-1-(3-Phenoxy-4-fluorphenyl)2-methylamino-, 1-(3-Phenoxy-4-fluorphenyl)-2-dimethylamino-, 1-(3-Phenoxy-4-fluorphenyl)-2-ethylamino-, 1-(3-Phenoxy-4-fluorphenyl)-2-diethylamino-, 1-(3-Phenoxy-4-fluorphenyl)-2-n-propylamino-, 1-(3-Phenoxy-4-fluorphenyl)-2-di-n-propylamino-, 1-(3-Phenoxy-4-fluorphenyl)-2-n-butylamino-, 1-(3-Phenoxy-4-fluor-

phenyl)-2-di-n-butylamino-, 1-(3-Phenoxy-4-fluorphenyl)-2-(ethyl-methylamino)-, 1-(3-Phenoxy-4-fluorphenyl)-2-(methyl-n-propylamino)-, 1-(3-Phenoxy-4-fluorphenyl)-2-(n-butyl-methylamino)-, 1-(3-Phenoxy-4-fluorphenyl)-2-(ethyl-n-propylamino)-, 1-(3-Phenoxy-4-fluorphenyl)-2-(n-butyl-ethylamino)-, 1-(3-Phenoxy-4-fluorphenyl)-2-(1-propen-3-yl-amino)-, 1-(3-Phenoxy-4-fluorphenyl)-2-cyclohexylamino-, 1-(3-Phenoxy-4-fluorphenyl)-2-cyclopropylamino-, 1-(3-Phenoxy-4-fluorphenyl)-2-n-dodecyl-amino-, 1-(3-Phenoxy-4-fluorphenyl)-2-piperazin-1-yl-, 1-(3-phenoxy-4-fluor-phenyl)-2-morpholin-4-yl-, 1-(3-Phenoxy-4-fluorphenyl)-2-imidazol-1-yl-, 1-(3-Phenoxy-4-fluorphenyl)-2-piperidin-1-yl-, 1-(3-Phenoxy-4-fluorphenyl)-2-(4-methyl-piperazin-1-yl)-, 1-(3-Phenoxy-4-fluorphenyl)-2-benzylamino-, 1-(3-Phenoxy-4-fluorphenyl)-2-(benzyl-methylamino)-, 1-(3-Phenoxy-phenyl)-2-dimethylamino-, 1-(3-Phenoxy-phenyl)-2-(methyl-n-propylamino)-ethanol und die entsprechenden Hydrochloride bzw. Hydrobromide.

Die neuen Aminoethanole der Formel (II b) erhält man nach dem unter (4) dargelegten Verfahren durch Umsetzung von Oxacyclopropanen der Formel IV b (oben) mit Aminen der Formel V (oben) in Gegenwart von Verdünnungsmitteln wie z.B. Wasser und/oder Alkoholen wie z.B. Ethanol bei Temperaturen zwischen 10 und 60°C.

Die Aufarbeitung kann nach üblichen Methoden durchgeführt werden, beispielsweise durch Abdestillieren des Lösungsmittels und anschließende Extraktion mit einem mit Wasser nicht mischbaren organischen Lösungsmittel. Die organische Phase wird getrocknet. filtriert und das Lösungmsittel wird unter vermindertem Druck sorgfältig abdestilliert, wobei man das Produkt als Rückstand erhält (siehe Herstellungsbeispiele).

Die bei Verfahren (2) außerdem als Ausgangsstoffe zu verwendenden Carbonsäuren sind durch Formel (III), die entsprechenden Säurechloride durch Formel (III a) definiert.

$R^5$ hat darin vorzugsweise die gleiche Bedeutung, wie sie bei der Definition der Verbindungen der Formel (I) als bevorzugt angegeben ist.

Die Carbonsäuren der Formel (III) sowie entsprechende Säurechloride der Formel (III a) sind bekannt (vergleiche US PS 3 996 244, 4 157 447, 3 962 458, 3 835 176; GB-PS 1 413 491 und 2 ooo 764; DE-OS 2 653 189 und P 3 128 444 [Le A 21 159]).

Die als Ausgangsprodukte für das Verfahren (4) einzusetzenden Oxacyclopropane sind durch die Formel (IV b) definiert. Vorzugsweise haben darin Y,m und Z die gleichen Bedeutungen, wie sie bei der Definition der entsprechenden Gruppen in Formel (II b) als bevorzugt angegeben sind.

Die Oxacyclopropane der Formel (IV b) sind neu. Als Beispiele für die neuen Verbindungen der Formel (IV b) seien genannt: 2-(4-Fluor-3-phenoxy-phenyl)-, 2-(3-Phenoxy-phenyl)-, 2-(3-Brom-4-fluor-phenyl)-, 2-(4-Brom-phenyl)-, 2-(4-Fluor-phenyl)-, -oxacyclopropan.

Die neuen Oxacyclopropane der Formel (IV b) erhält man nach dem unter (6) dargelegten Verfahren, indem man Aldehyde der Formel (VI) mit Trimethylsulfoniumhalogeniden der Formel (VII), gegebenenfalls in einer Schutzgasatmosphäre wie z.B. Stickstoff, gegebenenfalls in Gegenwart von Verdünnungsmitteln wie z.B. Dimethylsulfoxid und gegebenenfalls in Gegenwart von Säureakzeptoren wie z.B. Kalium-tert-butylat bei Temperaturen zwischen lo und 5o°C umsetzt.

Die Aufarbeitung der Reaktionsprodukte geschieht nach üblichen Methoden (siehe Herstellungsbeispiele).

Die für das Verfahren (4) ebenfalls einzusetzenden Amine sind durch die Formel (V) definiert. Vorzugsweise haben darin $R^3$ und $R^4$ die gleichen Bedeutungen, wie sie bei der Definition der entsprechenden Gruppen in Formel (I) als bevorzugt angegeben sind.

Die Amine der Formel (V) sind allgemein bekannte Verbindungen der organischen Chemie.

Die als Ausgangsstoffe für das Verfahren (6) einzusetzenden Aldehyde sind durch die Formel (VI) definiert. Vorzugsweise haben darin Y, Z und m die gleichen Bedeutungen, wie sie bei der Definition der entsprechenden Gruppen in Formel (II b) als bevorzugt angegeben sind.

Die Aldehyde der Formel (VI) sind allgemein bekannt (vergleiche z.B. US-PS 4 163 787, 3 835 176, 3 387 o37; DE-OS 2 7o9 264, 3 lo3 325 [Le A 2o 8ol], 2 o29 556; J.Gen.Chem.USSR 3o, (196o), 31o3; Bull.Soc.Chim.France 1955, 1594 ibid. 1962, 254-262; J.Org. Chem. 37, (1972), 673; J.Med.Chem. 16 (1973), 1399).

Die außerdem als Ausgangsstoffe für das Verfahren (6) einzusetzenden Trimethylsulfoniumhalogenide sind durch die Formel (VII) definiert. Vorzugsweise steht Hal darin für Chlor, Brom oder Iod.
Als Beispiele für die Verbindungen der Formel (VII) seien genannt: Trimethylsulfonium-chlorid, -bromid bzw. -iodid.

Die Trimethylsulfoniumhalogenide der Formel (VII) sind allgemein bekannte Verbindungen der organischen Chemie.

Die Wirkstoffe eignen sich bei guter Pflanzenverträglichkeit und günstiger Warmblütertoxizität zur Bekämpfung von tierischen Schädlingen, insbesondere Insekten, Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims,

Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-,Azol-u.Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen.

Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

## Herstellungsbeispiele

**Beispiel 1:**

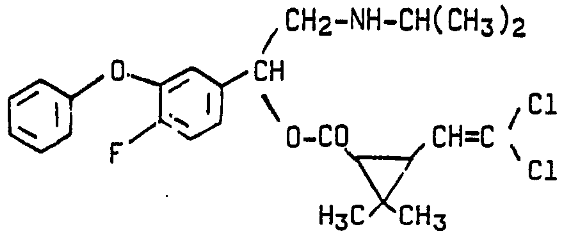

4,9 g (o,ol7 Mol) 1-(4-Fluor-3-phenoxy-phenyl)-2-(2-propylamino)-ethanol werden in 5o ml Toluol gelöst und bei lo°C Chlorwasserstoff-Gas bis zur Sättigung eingeleitet. Anschließend wird das Lösungsmittel im Vakuum bis auf $^1/_{10}$ des ursprünglichen Volumens abgezogen. Der Rückstand wird mit 4 g (o,ol7 Mol) cis/trans-3-Dichlorvinyl-2,2-dimethyl-cyclopropancarbonsäurechlorid versetzt und unter Rühren 5 Stunden auf 1lo°C erhitzt. Nach beendeter Reaktionszeit wird das Reaktionsgemisch in 15o ml Wasser gegossen, durch Zugabe von Natriumcarbonat-Lösung auf pH 7 eingestellt und anschließend mit loo ml Toluol extrahiert. Die organische Phase wird über Magnesiumsulfat getrocknet, das organische Lösungsmittel im Wasserstrahlvakuum abdestilliert und die letzten Lösungsmittelreste durch kurzes Andestillieren bei 7o°C/2 mm Hg Badtemperatur entfernt. Man erhält 6 g (73,5% der Theorie) cis/-trans-3-Dichlor-vinyl-2,2-dimethyl-cyclopropancarbonsäure-1-(4-fluor-3-phenoxy -phenyl)-2-(2-propylamino)-ethylester als zähes gelbes Oel mit dem Brechungsindex $n_D^{20}$: 1,548o.

Die Struktur wird durch das $^1$H-NMR-Spektrum und IR-Spektrum bestätigt.

$^1$H-NMR-Spektrum/$CDCl_3$ $\tau$(ppm):

Benzyl - H: 5,55 - 5,85 (m/1 H)

IR-Spektrum/Film ($cm^{-1}$):

$>C=O$ : 173o

Beispiel 2

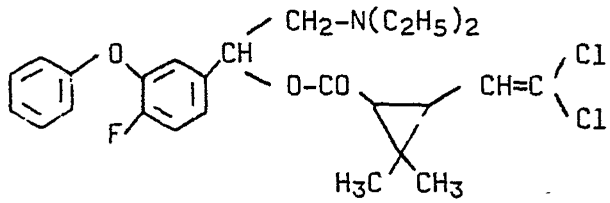

6,08 g (0,02 Mol) 1-(4-Fluor-3-phenoxy-phenyl)-2-diethylamino-ethanol und 2 g Triethylamin werden in 100 ml Toluol gelöst und 4,7 g (0,02 Mol) cis/trans-3-Dichlorvinyl-2,2-dimethylcyclopropancarbonsäure, gelöst in 20 ml Toluol bei 20°C unter Rühren zugetropft. Anschließend wird weitere 3 Stunden bei 25-30°C gerührt. Das Reaktionsgemisch wird in 150 ml Wasser gegossen, mit Natriumcarbonat-Lösung pH 7 eingestellt und dann mit 150 ml Toluol extrahiert. Die Toluolphase wird über Magnesiumsulfat getrocknet und das Lösungsmittel im Wasserstrahlvakuum abdestilliert. Letzte Lösungsmittelreste werden durch kurzes Andestillierne bei 60°C/ 2 mm Hg Badtemperatur entfernt. Man erhält 4 g (40,5% der Theorie) cis/trans-3-Dichlorvinyl-2,2-dimethyl-cyclopropancarbonsäure-1-(4-fluor-3-phenoxy-phenyl)-2-diethylamino-ethylester als zähes Oel. Die Struktur wird durch das $^1$H-NMR-Spektrum und IR-Spektrum bestätigt.

$^1$H-NMR-Spektrum/$CDCl_3$/$\tau$(ppm):
Benzyl-H: 5,9-6,2 (m/1 H)

IR-Spektrum/($cm^{-1}$):
$>$C=O : 1730

Analog Beispiel (1) und (2) lassen sich folgende Verbindungen der Formel (I) herstellen:

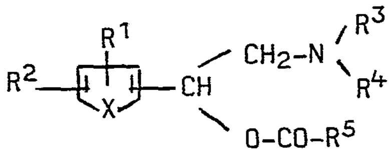 (I)

(3) 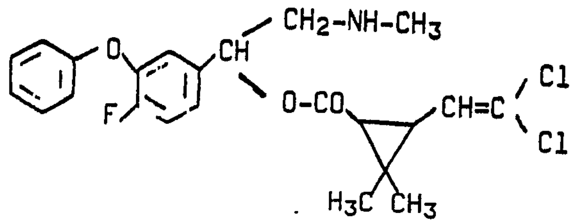

55% der Theorie ; zähes Oel

(4) 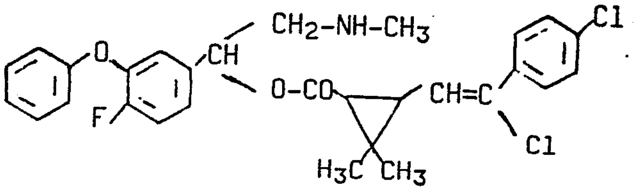

6o% der Theorie ; zähes Oel

(5) 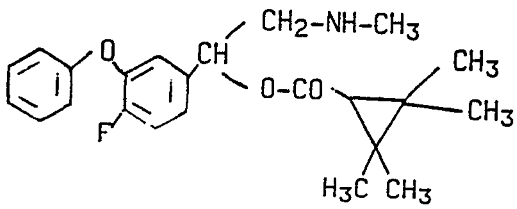

65% der Theorie ; zähes Oel

$^1$H-NMR/$\tau$(ppm):

Benzyl-H: 4,o-4,35 (m/1H)

(6) 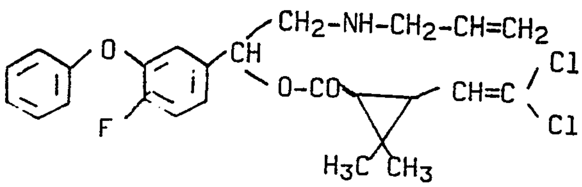

73% der Theorie ; zähes Oel

(7) 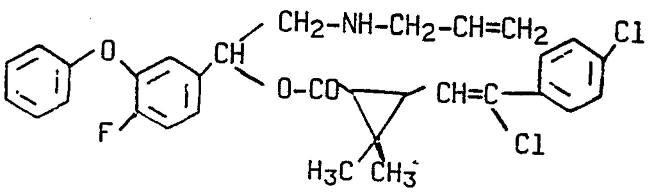

63% der Theorie ; zähes Oel

(8) 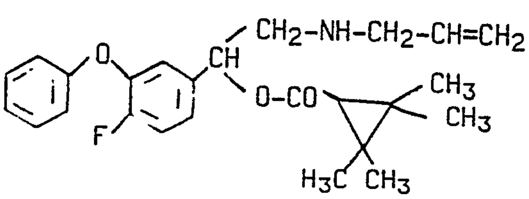

61% der Theorie ; zähes Oel

$^1$H-NMR/$\tau$(ppm):

Benzyl-H: 4,o-4,3 (m/1 H)

(9) 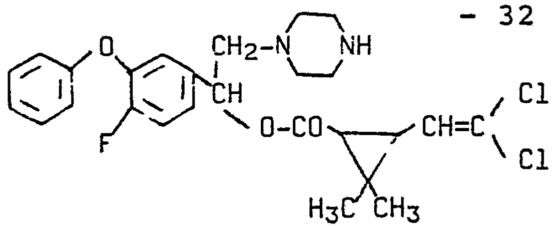

59% der Theorie ; zähes Oel

$^1$H-NMR/$\tau$(ppm)

Benzyl-H: 3,85-4,15 (m/1 H)

(1o) 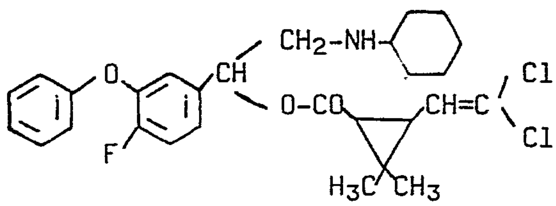

77% der Theorie ; zähes Oel

(11) 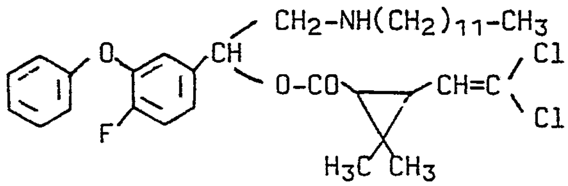

78% der Theorie ; zähes Oel

(12) 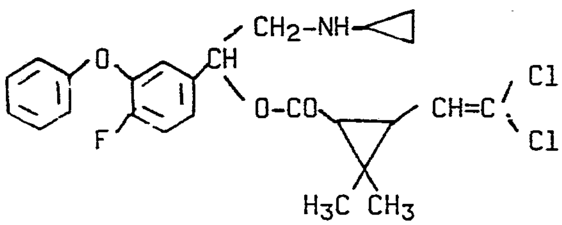

52% der Theorie ; zähes Oel

(13) 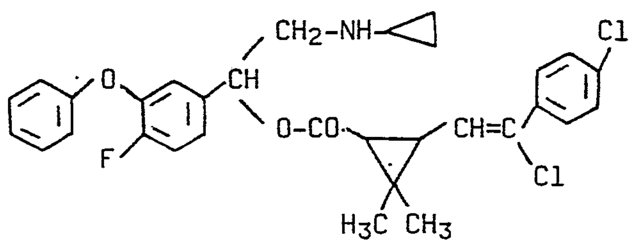

63% der Theorie ; zähes Oel

(14) 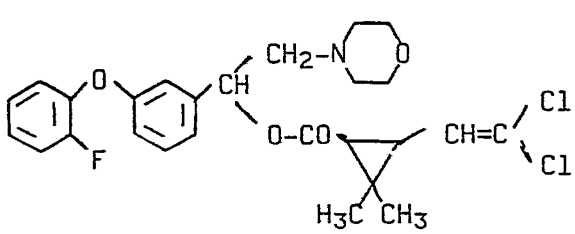

79% der Theorie ; zähes Oel

$^1$H-NMR/$\tau$(ppm)

Benzyl-H: 3,95-4,3 (m/1 H)

(15) 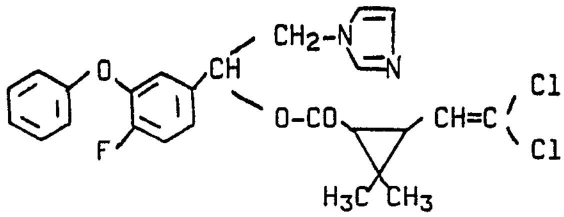

82% der Theorie ; zähes Oel

$^{1}$H-NMR/τ(ppm):

Benzyl-H: 4,o-4,3 (m/1 H)

(16) 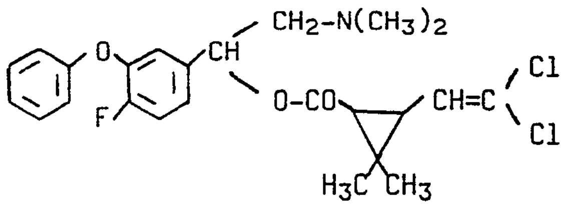

64% der Theorie ; $n_D^{20}$:1,5498

(17) 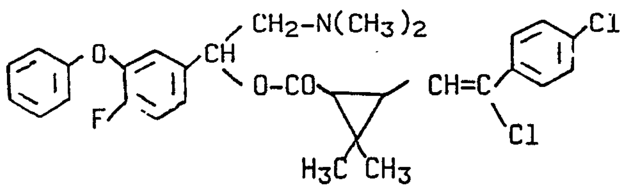

74% der Theorie ; zähes Oel

$^{1}$H-NMR/τ(ppm):

Benzyl-H: 3,95-4,3 (m/1 H)

(18) 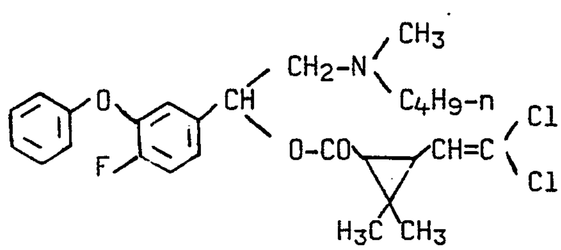

79% der Theorie ; zähes Oel

(19) 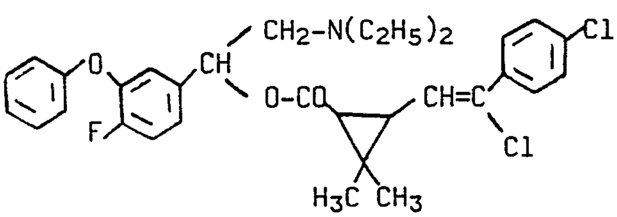

79% der Theorie ; zähes Oel

$^{1}$H-NMR/ (ppm)

Benzyl-H: 4,o-4,3 (m/1 H)

(2o) 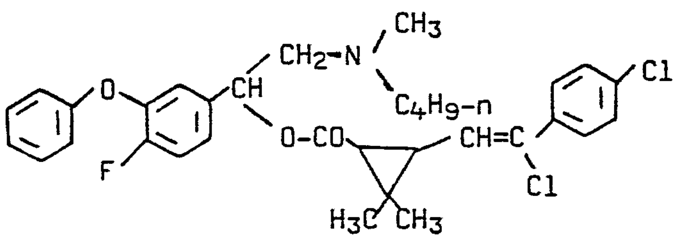

68% der Theorie ; zähes Oel

(21) 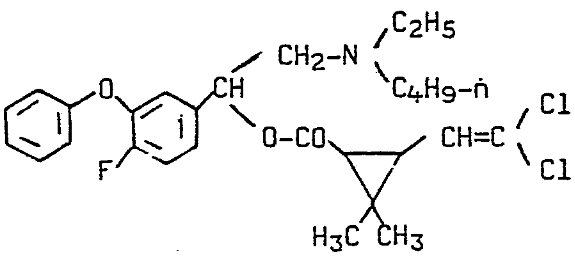

67% der Theorie ; zähes Oel

$^1H$-NMR/τ(ppm)

Benzyl-H: 4,o-4,3 (m/1H)

(22) 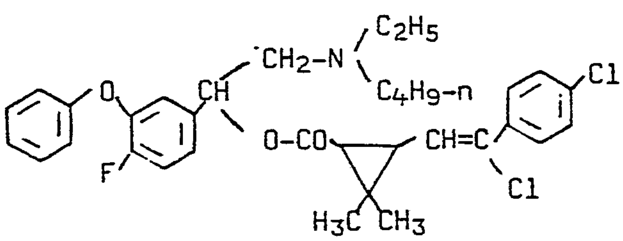

67% der Theorie ; zähes Oel

$^1H$-NMR/τ(ppm)

Benzyl-H: 4,1-4,4 (m/1 H)

(23) 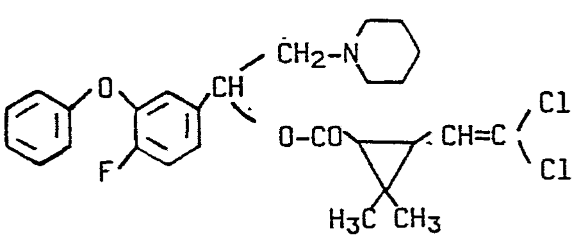

69% der Theorie ; zähes Oel

$^1H$-NMR/ (ppm)

Benzyl-H: 4,o5-4,35 (m/1 H)

(24) 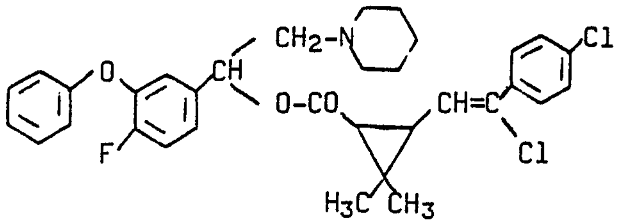

77% der Theorie ; zähes Oel

<u>$^1$H-NMR/τ(ppm)</u>

Benzyl-H: 4,o5-4,28 (m/1H)

(25) 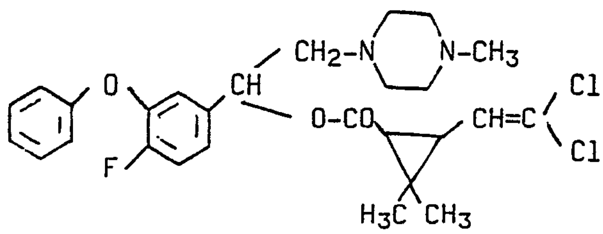

48% der Theorie ; zähes Oel

<u>$^1$H-NMR/τ(ppm)</u>

Benzyl-H: 4,o5-4,3 (m/1H)

(26) 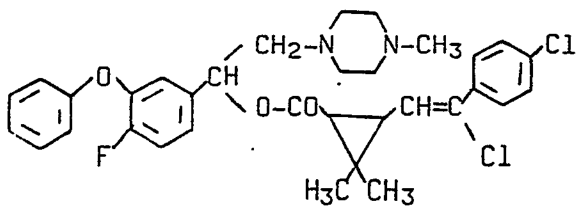

5o% der Theorie ; zähes Oel

(27) 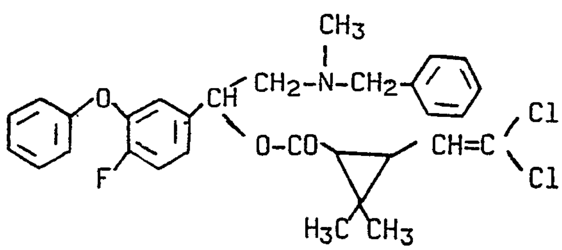

74% der Theorie ; zähes Oel

<u>$^1$H-NMR/τ(ppm)</u>

-CH : 4,o-4,3 (m/1 H)
O-

(28) 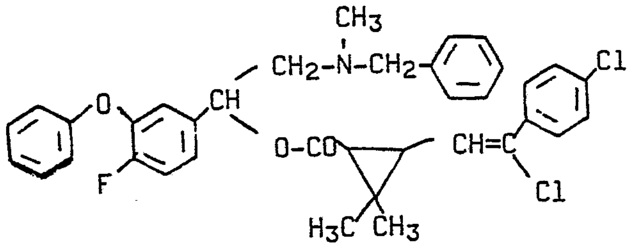 71% der Theorie ; zähes Oel

(29) 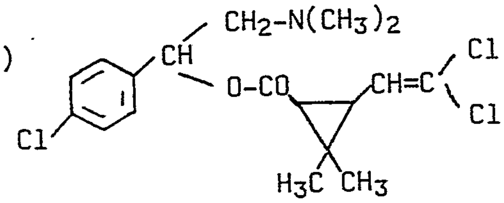 zähes Oel

(3o) 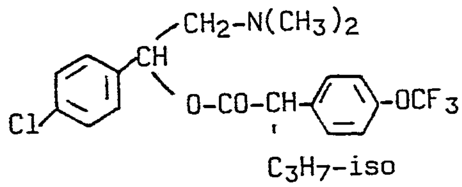 zähes Oel

(31) 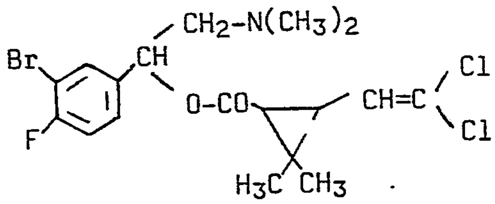 zähes Oel

Ausgangsprodukte

Beispiel (a)

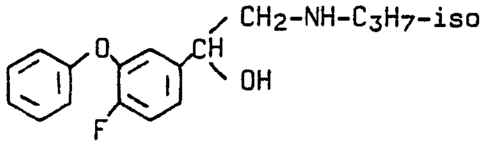

Eine Mischung von 11,5 g (o,o5 Mol) 4-Fluor-3-phenoxy-styryloxid und 15o ml wäßrige 7o%ige iso-Propylaminlösung wird bei 2o-25°C 16 Stunden intensiv gerührt. Anschließend wird das Wasser im Vakuum abdestilliert, der Rückstand in Methylenchlorid gelöst und über Magnesium-sulfat getrocknet. Das organische Lösungsmittel wird dann im Vakuum abgezogen. Der ölige Rückstand, der durch sogenanntes Andestillieren bei 7o°C Badtemperatur und 3 mm Hg von letzten Lösungsmittelresten befreit wird, erstarrt nach kurzer Zeit zu farblosen Kristallen. Man erhält lo,2 g (7o,5% der Theorie) 1-(4-Fluor-3-phenoxy-phenyl)-2-(2-propylamino)-ethanol mit einem Schmelzpunkt von 99-loo°C. Die Struktur wird durch das $^{1}$H-NMR-Spektrum bestätigt:

$^{1}$H-NMR/$CDCl_3$/$\tau$(ppm)
Benzyl-H: 5,3-5,58 (m/1 H)

Analog Beispiel (a) lassen sich folgende Verbindungen der Formel (II) herstellen:

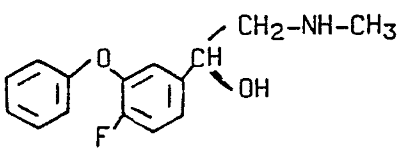

77% der Theorie; zähes Oel

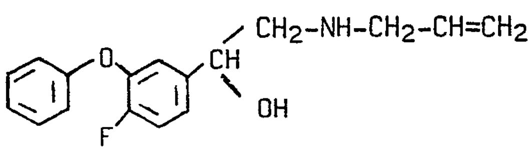

76% der Theorie; Schmelzpunkt 89-9o°C

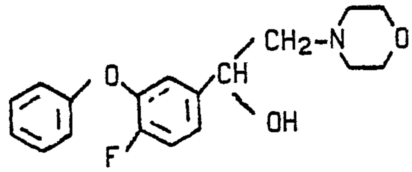

92% der Theorie
Schmelzpunkt:
110-111°C

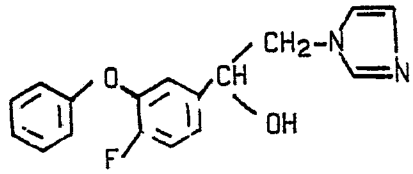

34% der Theorie
Schmelzpunkt:
117-118°C

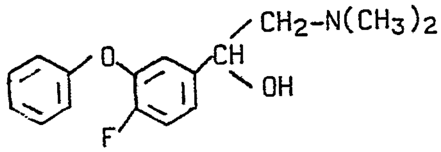

94% der Theorie
Brechungsindex:
$n_D^{20}$: 1,5593

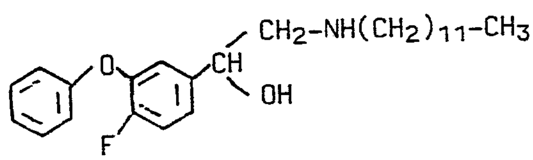

63% der Theorie
Schmelzpunkt:
50-52°C

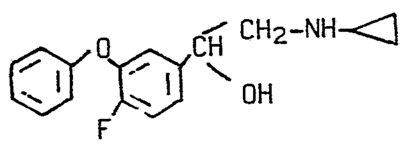

84% der Theorie
Schmelzpunkt:
76-77°C

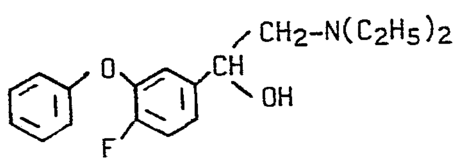

69% der Theorie;
zähes Oel

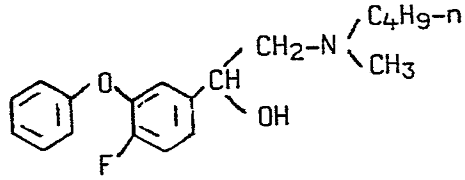

57% der Theorie;
zähes Oel

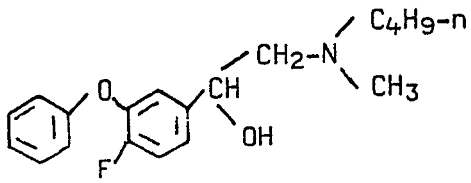

60% der Theorie ;
zähes Oel

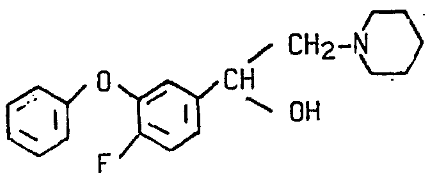

76% der Theorie ;
zähes Oel

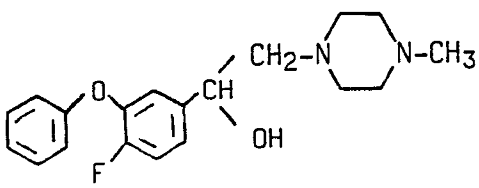

24% der Theorie ;
zähes Oel

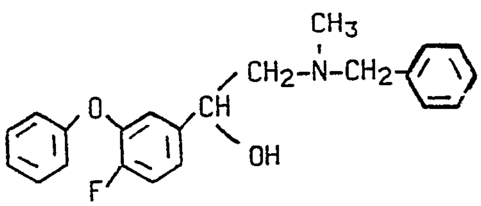

63% der Theorie ;
sehr zähes Oel

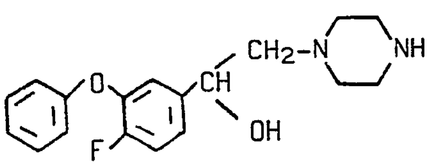

9% der Theorie
Schmelzpunkt:
136-138°C

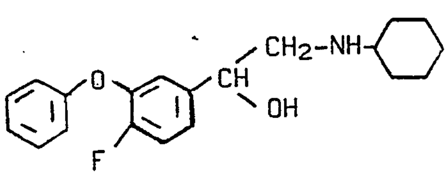

52% der Theorie
Schmelzpunkt:
1o9°C

Beispiel (b)

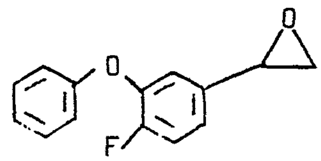

Zu einer Mischung von 12o ml Dimethylsulfoxid, 43,4 g (o,2 Mol) 4-Fluor-3-phenoxy-benzaldehyd und 61,2 g (o,3 Mol) Trimethylsulfoniumiodid werden unter Stickstoff und gutem Rühren 28 g (o,25 Mol) Kalium-tert-butylat gelöst in 12o ml Dimethylsulfoxid bei 2o°C langsam zugetropft. Anschließend wird noch 3o Minuten bei 2o-25°C nachgerührt. Zum Reaktionsgemisch läßt man dann unter Eiskühlung 6oo ml Wasser zutropfen und extrahiert dann das Gemisch 3 mal mit je 4oo ml Diethylether. Die vereinigten Etherphasen werden anschließend 3 mal mit je 4oo ml Wasser ausgeschüttelt und dann über Magnesiumsulfat getrocknet. Das organische Lösungsmittel wird im Vakuum abgezogen und der ölige Rückstand im Vakuum destilliert. Man erhält 18,4 g (4o% der Theorie) 4-Fluor-3-phenoxy-styryloxid als leicht gelbliche Flüssigkeit mit einem Siedepunkt von 17o-175°C/1o mm Hg.

Analog Beispiel (b) werden die übrigen Oxacylalkane der Formel (IV) erhalten.

**Beispiel A**

Laphygma-Test

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenflaters (Laphygma frugiperda) besetzt, solange die Blätter noch feucht sind.

Nach der gewünschten Zeit wird die Abtötung in % bestimmt. Dabei bedeutet 100 %,daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 16, 17, 18, 27, 2, 19, 21, 23, 14, 25, 26, 3 und 1.

Beispiel B

Test mit Boophilus microplus resistent

Lösungsmittel: 35 Gewichtsteile Ethylenglykolmonomethylether
35 Gewichtsteile Nonylphenolpolyglycolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

lo adulte Boophilus microplus res. werden in die zu testende Wirkstoffzubereitung 1 Minute getaucht. Nach Ueberführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik: 17, 2, 19, 2o, 22, 24 und 28.

Patentansprüche:

1. Aminoalkylester der Formel (I)

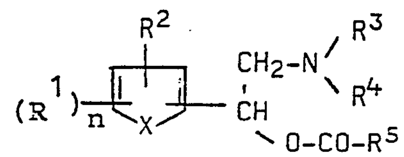

(I)

in welcher

$R^1$ und $R^2$ gleich oder verschieden sind und für Wasserstoff, gegebenenfalls substituierte Reste aus der Reihe Alkyl, Alkenyl, Alkinyl, Alkoxy, Alkylthio, Aryl, Aralkyl und Aryloxy sowie für Halogen, Nitro, und Cyano stehen, oder

$R^1$ und $R^2$ zusammen für gegebenenfalls substituiertes Alkylen oder Alkylendioxy stehen,

n für 1 oder 2 steht,

$R^3$ und $R^4$ gleich oder verschieden sind und für Wasserstoff oder gegebenenfalls substituierte Reste aus der Reihe Alkyl, Alkenyl, Cycloalkyl, Aryl oder Aralkyl stehen, oder

$R^3$ und $R^4$ zusammen mit dem Stickstoff an den sie gebunden sind, für gegebenenfalls substituiertes Pyrrolidin-1-yl, Piperidin-1-yl, Morpholin-4-yl, Imidazol-1-yl, Pyrazol-1-yl, Pyrimidin-1-yl, Imidazolidin-1-yl, Piperazin-1-yl oder Pyrazolidin-1-yl stehen,

$R^5$ für einen in der Säurekomponente von Pyrethroiden verwendbaren Rest steht, und

X für -O-, -S-, -NH-, -CH=CH-, -CF=CF-, CH=N- steht.

2. Verfahren zur Herstellung der neuen Verbindungen der Formel I

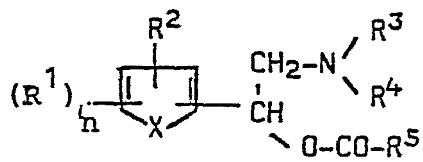

(I)

in welcher

$R^1$ und $R^2$ gleich oder verschieden sind und für Wasserstoff, gegebenenfalls substituierte Reste aus der Reihe Alkyl, Alkenyl, Alkinyl, Alkoxy, Alkylthio, Aryl, Aralkyl und Aryloxy sowie für Halogen, Nitro und Cyano stehen, oder

$R^1$ und $R^2$ zusammen für gegebenenfalls substituiertes Alkylen oder Alkylendioxy stehen,

n für 1 oder 2 steht,

$R^3$ und $R^4$ gleich oder verschieden sind und für Wasserstoff oder gegebenenfalls substituierte Reste aus der Reihe Alkyl, Alkenyl, Cycloalkyl, Aryl und Aralkyl stehen, oder

$R^3$ und $R^4$ zusammen mit dem Stickstoffatom, an das sie gebunden sind für gegebenenfalls substituiertes Pyrrolidin-1-yl, Piperidin-1-yl, Morpholin-4-yl, Imidazol-1-yl, Pyrazol-1-yl, Pyrimidin-1-yl, Imidazolin-1-yl, Piperazin-1-yl oder Pyrazolidin-1-yl stehen

$R^5$ für einen in der Säurekomponente von Pyrethroiden verwendbaren Rest steht und

X für -O-, -S-, -NH-, -CH=CH-, -CF=CF-, -CH=N- steht,

dadurch gekennzeichnet, daß man Aminoethanole der Formel II

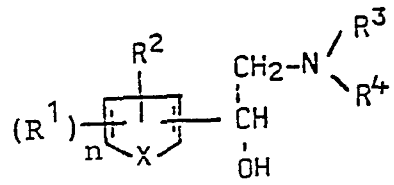

(II)

in welcher
$R^1$, $R^2$, $R^3$, $R^4$ und X die oben angegebenen Bedeutungen haben,

oder deren Salze,

mit Carbonsäuren der Formel III

$$R^5COOH$$ (III)

in welcher

$R^5$ die oben angegebene Bedeutung hat,

oder mit reaktionsfähigen Derivaten derselben, gegebenenfalls in Gegenwart von Säureakzeptoren, gegebenenfalls in Gegenwart von Katalysatoren und gegebenenfalls in Gegenwart von Verdünnungsmitteln umsetzt.

3. Aminoethanole der Formel II b

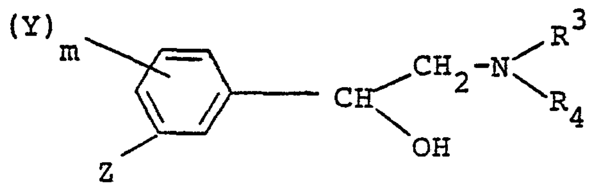

(II b)

in welcher

Z für Fluor, Brom oder gegebenenfalls substituiertes Aryloxy steht,

Y für Wasserstoff oder Fluor

m für 1-4 steht,

$R^3$ und $R^4$ die oben angegebene Bedeutung haben.

4. Verfahren zur Herstellung der neuen Verbindungen der Formel (II b) gemäß Anspruch 1, dadurch gekennzeichnet, daß man Oxacylcyclopropane der Formel IV b

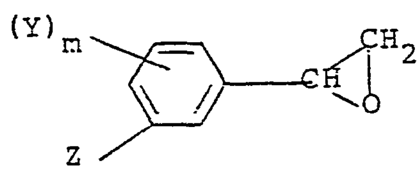

(IV b)

in welcher

Y, Z und m die oben angegebenen Bedeutungen haben,

mit Aminen der Formel V

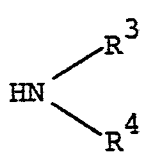

(V)

in welcher

$R^3$ und $R^4$ die oben angegebene Bedeutung haben,

gegebenenfalls in Gegenwart von Verdünnungsmitteln und/oder gegebenenfalls in Gegenwart eines Katalysators bei Temperaturen zwischen 0 und 100°C umsetzt;

5. Oxacyclopropane der Formel IV b

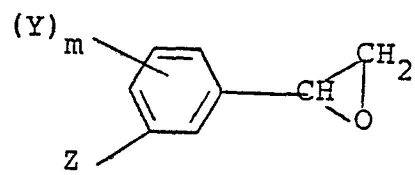

(IV b)

in welcher

Z für Fluor, Brom oder gegebenenfalls substituiertes Aryloxy steht,
Y für Wasserstoff oder Fluor steht,
m für 1-4 steht.

6. Verfahren zur Herstellung der Oxacyclopropane der Formel (IV b) gemäß Anspruch 5, dadurch gekennzeichnet, daß man Aldehyde der Formel VI

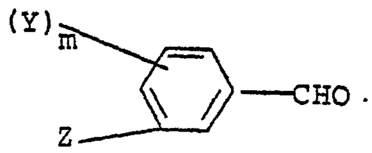

(VI)

in welcher

Z, Y und m die oben angegebenen Bedeutungen haben,

mit Trimethylsulfoniumhalogeniden der Formel VII

$$(H_3C)_3S^{\oplus}\,Hal^{\ominus} \qquad \text{(VII)}$$

in welcher

Hal für Chlor, Brom oder Iod steht,

gegebenenfalls in einer Schutzgasatmosphäre, gegebenenfalls in Gegenwart von Säureakzeptoren und gegebenenfalls in Gegenwart von Verdünnungsmitteln bei Temperaturen zwischen 10 und 50°C umsetzt.

7. Schädlingsbekämpfungsmittel, gekennzeichnet durch einen Gehalt an mindestens einem Aminoalkylester der Formel (I).

8. Verwendung von Aminoalkylester der Formel (I) zur Bekämpfung von Schädlingen.

9. Verfahren zur Bekämpfung von Schädlingen, dadurch gekennzeichnet, daß man Aminoalkylester der Formel (I) auf Schädlinge und/oder ihren Lebensraum einwirken läßt.

10. Verfahren zur Herstellung von Schädlingsbekämpfungsmitteln, dadurch gekennzeichnet, daß man Aminoalkylester der Formel (I) mit Streckmitteln und/oder oberflächenaktiven Mitteln vermischt.